(19) **Europäisches Patentamt / European Patent Office / Office européen des brevets**

(11) **EP 4 741 372 A1**

(12) **EUROPEAN PATENT APPLICATION**
published in accordance with Art. 153(4) EPC

(43) Date of publication:
**13.05.2026 Bulletin 2026/20**

(21) Application number: **24835299.9**

(22) Date of filing: **28.06.2024**

(51) International Patent Classification (IPC):
**C07C 257/12** $^{(2006.01)}$

(52) Cooperative Patent Classification (CPC):
Y02E 10/549

(86) International application number:
**PCT/CN2024/102626**

(87) International publication number:
**WO 2025/007818 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.07.2023 CN 202310812876**

(71) Applicant: **Contemporary Amperex Technology Co., Limited**
**Ningde, Fujian 352100 (CN)**

(72) Inventors:
• **LUAN, Bo**
  **Ningde, Fujian 352100 (CN)**
• **LIANG, Weifeng**
  **Ningde, Fujian 352100 (CN)**
• **SHI, Ruoxuan**
  **Ningde, Fujian 352100 (CN)**
• **CHANG, Yuhang**
  **Ningde, Fujian 352100 (CN)**
• **GUO, Yongsheng**
  **Ningde, Fujian 352100 (CN)**
• **CHEN, Guodong**
  **Ningde, Fujian 352100 (CN)**

(74) Representative: **Ran, Handong**
**Maucher Jenkins**
**Seventh Floor Offices**
**Artillery House**
**11-19 Artillery Row**
**London SW1P 1RT (GB)**

(54) **USE OF ADDITIVE, FORMAMIDINIUM IODIDE AND PREPARATION METHOD THEREFOR, PEROVSKITE SOLAR CELL, SOLAR POWER GENERATION DEVICE, AND ELECTRIC DEVICE**

(57)     The present application discloses a use of an additive, formamidinium iodide and a preparation method therefor, a perovskite solar cell, a solar power generation device, and an electric device. The additive comprises a reducing agent. By incorporating the reducing agent in the additive and using the additive to prepare formamidinium iodide, generation of elemental iodine in the preparation process of formamidinium iodide can be reduced; in addition, iodine which has been oxidized to elemental iodine can be reduced to iodine anions so as to generate formamidinium iodide. The stability of formamidinium iodide is improved, and thus the stability and efficiency of perovskite cells are improved.

EP 4 741 372 A1

# EP 4 741 372 A1

**Description**

## CROSS-REFERENCE TO RELATED APPLICATIONS

The present application claims priority to Chinese Patent Application No. 202310812876.5, filed with the China Patent Office on July 04, 2023 and entitled "APPLICATION OF ADDITIVE, FORMAMIDINIUM IODIDE AND PREPARATION METHOD THEREFOR, PEROVSKITE SOLAR CELL, SOLAR POWER GENERATION APPARATUS, AND ELECTRICAL APPARATUS", which is incorporated herein by reference in its entirety.

## TECHNICAL FIELD

The present application relates to the technical field of batteries, and specifically relates to application of an additive, formamidinium iodide and a preparation method therefor, a perovskite solar cell, a solar power generation apparatus, and an electrical apparatus.

## BACKGROUND

Formamidinium iodide is one of the important raw materials for perovskite solar cells. With the development of the perovskite solar cells, the demand for formamidinium iodide will continue to increase. In current preparation process, bulk-produced formamidinium iodide products have iodine precipitation and surface yellowing, and the color deepens as the storage time increases. Iodine precipitated from formamidinium iodide will be treated as a carrier recombination center in subsequent preparation of formamidinium iodide perovskite films, thereby reducing the stability and efficiency of the perovskite solar cells.

## SUMMARY OF THE INVENTION

The present application is proposed in view of the above problems, and provides application of an additive in preparation of formamidinium iodide, aiming to reduce the precipitation of elemental iodine in a preparation process of formamidinium iodide, thereby improving the stability and efficiency of a perovskite cell.

In order to achieve the above purpose, the embodiments of the present application provide application of an additive, formamidinium iodide and a preparation method therefor, a perovskite solar cell, a solar power generation apparatus, and an electrical apparatus.

In a first aspect, the embodiment of the present application provides the application of the additive in preparation of formamidinium iodide, and the additive includes a reducing agent.

According to the technical solution in the embodiment of the present application, by incorporating the reducing agent in the additive and using the additive to prepare formamidinium iodide, the generation of elemental iodine in the preparation process of formamidinium iodide can be reduced; in addition, iodine which is oxidized to elemental iodine can be reduced to iodine anions so as to generate formamidinium iodide, therefore, the stability of formamidinium iodide is improved, and then the stability and efficiency of the perovskite cell are improved.

In any embodiment, the reducing agent includes a compound containing at least one of elements N, O, S and P. When the reducing agent including the compound containing at least one of elements N, O, S and P is used for preparing formamidinium iodide, the generation of the elemental iodine in the preparation process of formamidinium iodide can be reduced, and moreover, iodine which is oxidized to elemental iodine can be reduced to iodine anions so as to generate formamidinium iodide, therefore, the stability of formamidinium iodide is improved; moreover, when the prepared formamidinium iodide is applied to the perovskite cell, in a perovskite precursor solution, and in the presence of the reducing agent including the compound containing at least one of elements N, O, S and P, the reducing agent will be complexed with iodide, thus the solvent will be quickly removed, perovskite can be quickly nucleated and slowly crystallized, and then the stability and efficiency of the perovskite cell are further improved. In absence of the reducing agent including the compound containing at least one of elements N, O, S and P, iodide will be complexed with DMSO in the solvent, so that the crystallization rate of perovskite is increased, which does not facilitate nucleation and solvent volatilization.

In any embodiment, the reducing agent includes at least one of hypophosphorous acid, red phosphorus, sulfurous acid, L-ascorbic acid, sodium thiosulfate, sodium hypophosphite, oxalic acid, hydrosulfuric acid and formamidinium sulfinic acid. By using at least one of the reducing agents, the precipitation of elemental iodine in the preparation process of formamidinium iodide can be further reduced, thus the perovskite nucleation rate is increased, the perovskite crystallization rate is decreased, the solvent volatilization rate is increased, and then the stability and efficiency of the perovskite cell are improved. In some embodiments of the present application, the reducing agent can only include one of hypophosphorous acid, red phosphorus, sulfurous acid, L-ascorbic acid, sodium thiosulfate, sodium hypophosphite, oxalic acid, hydrosulfuric acid and formamidinium sulfinic acid, and in other embodiments of the present application, two or more of them can be included at the same time.

In a second aspect, the embodiment of the present application provides the preparation method for formamidinium iodide, and the additive in the first aspect is used. In some embodiments of the preparation method for formamidinium iodide in the present application, the steps of using the additive are not limited, the additive can be pretreated in raw materials, or the additive can be added in the reaction process to reduce the generation of elemental iodine.

In any embodiment, the preparation method for formamidinium iodide includes the following steps:

mixing hydroiodic acid with the additive, cooling and storing to obtain a hydroiodic acid pretreatment solution; and mixing a formamidinium acetate solution with the hydroiodic acid pretreatment solution, crystallizing, washing and drying to obtain formamidinium iodide.

The additive is added into hydroiodic acid, and cooled and stored to obtain the hydroiodic acid pretreatment solution; the reducing agent is added into the hydroiodic acid, the reducing agent will react with the elemental iodine in the hydroiodic acid firstly to reduce the elemental iodine to hydroiodic acid; and the reducing agent is oxidized, so that the content of the elemental iodine in the hydroiodic acid pretreatment solution is reduced. Then the hydroiodic acid pretreatment solution reacts with formamidinium acetate, and a main product of the reaction is formamidinium iodide, and therefore, the content of the elemental iodine in the formamidinium iodide is reduced. In addition, the cooling and storing can further reduce the generation of the elemental iodine.

In any embodiment, in the step of mixing hydroiodic acid with the additive, cooling and storing to obtain the hydroiodic acid pretreatment solution,
the mass ratio of the additive in the hydroiodic acid pretreatment solution is 0.1%-10%, and the mass ratio of the additive in the hydroiodic acid pretreatment solution is within this range, so that the inhibition effect on the elemental iodine can be improved; the reducing agent is provided to react with the elemental iodine, and meanwhile, the reaction between the reducing agent and the formamidinium acetate is reduced, thus side reaction products can be reduced, and impurities can be reduced; and when the prepared formamidinium iodide is used for preparing the perovskite cell, the stability and the cycle performance of the perovskite cell are facilitated, and the cost is reduced. Optionally, the mass ratio of the additive in the hydroiodic acid pretreatment solution is 0.5%-5%.

In any embodiment, in the step of mixing hydroiodic acid with the additive, cooling and storing to obtain the hydroiodic acid pretreatment solution,

the cooling and storing temperature is 0-10°C, which can reduce icing, and is conducive to the reaction between the reducing agent and the elemental iodine, thereby reducing the precipitation of the elemental iodine; optionally, the cooling and storing temperature is 0-5°C; and/or,
the cooling and storing time is 24-72 h, which can improve the reaction efficiency of the reducing agent and the elemental iodine, as well as improving the production efficiency; and optionally, the cooling and storing time is 36-60 h.

In any embodiment, the ratio of the amount of substance of formamidinium acetate to hydroiodic acid is 1:(0.9-1.2), appropriate ratio of the amount of substance can ensure sufficient reaction, and reduce the residues of reaction raw materials. Optionally, the ratio of the amount of substance of the formamidinium acetate solution to hydroiodic acid is 1:(1.05-1.1).

In any embodiment, in the step of mixing the formamidinium acetate solution with the hydroiodic acid pretreatment solution, crystallizing, washing and drying to obtain formamidinium iodide,

the mixing temperature is 0-35°C, and at this temperature, the yield of formamidinium iodide can be increased, and meanwhile icing is reduced; optionally, the mixing temperature is 0-5°C; and/or,
the mixing time is 2-8 h, at such time, hydroiodic acid and formamidinium acetate can react sufficiently, thereby increasing the yield of formamidinium iodide, making reaction more sufficient, and reducing the influence on the production efficiency; optionally, the mixing time is 3-5 h; and/or,
the crystallization temperature is 0-35°C, which can increase the crystallization rate of formamidinium iodide, and reduce icing; optionally, the crystallization temperature is 0-10°C; and/or,
the drying mode includes vacuum drying, in which, the drying pressure is 0 bar to -1 bar, contact between formamidinium iodide and oxygen can be reduced by vacuum drying, and then the generation of elemental iodine is reduced; optionally, the drying pressure is -0.8 bar to -1 bar; and/or,
the drying temperature is 40-80°C, which is conducive to volatilization of the solvent, and reduces the risk of scald in the preparation process; optionally, the drying temperature is 60-70°C; and/or,
the drying time is 6-15 h, which can reduce the residues of the solvent, and improve the production efficiency; and optionally, the drying time is 8-12 h.

In any embodiment, the step of mixing the formamidinium acetate solution with the hydroiodic acid pretreatment solution, crystallizing, washing and drying to obtain formamidinium iodide includes:

mixing the formamidinium acetate solution with the hydroiodic acid pretreatment solution to obtain a mixed solution;
evaporating the mixed solution, and removing the solvent to obtain a formamidinium iodide crude extract; and
mixing the formamidinium iodide crude extract with a solvent, heating, recrystallizing, washing and drying to obtain formamidinium iodide.

By recrystallizing the formamidinium iodide crude extract, impurity residues in the formamidinium iodide crude extract can be further removed, and therefore, the purity of formamidinium iodide is improved. In some embodiments of the present application, recrystallization can be one-time recrystallization or multi-recrystallization according to different requirements.

In any embodiment, in the step of mixing the formamidinium iodide crude extract with a solvent, crystallizing, washing and drying to obtain formamidinium iodide,

the solvent includes ethanol, and the ethanol is used as the solvent and is conveniently removed, so that the impurities in formamidinium iodide can be decreased; and/or,
the heating temperature is 30-70°C, which is conducive to the dissolution of formamidinium iodide, and the volatilization speed of ethanol is controlled, thereby facilitating recrystallization; optionally, the heating temperature is 40-50°C; and/or
the recrystallization temperature is 0-35°C, which can increase the crystallization rate of formamidinium iodide and reduce icing; and optionally, the recrystallization temperature is 0-5°C.

In a third aspect, the embodiment of the present application provides formamidinium iodide which is prepared by the preparation method for formamidinium iodide in the second aspect of the present application.

In a fourth aspect, the embodiment of the present application provides a perovskite solar cell which includes formamidinium iodide in the third aspect of the present application.

In a fifth aspect, the embodiment of the present application provides a solar power generation apparatus which includes the perovskite solar cell in the fourth aspect of the present application.

In a sixth aspect, the embodiment of the present application provides an electrical apparatus which includes the solar power generation apparatus in the fifth aspect of the present application.

## DETAILED DESCRIPTION

The embodiments of an application of an additive, formamidinium iodide and a preparation method therefor, a perovskite solar cell, a solar power generation apparatus, and an electrical apparatus of the present application will be specifically disclosed below. However, unnecessary detailed explanations may be omitted. For example, there are cases where detailed descriptions of well-known items and repeated descriptions of actually identical structures are omitted. This is to avoid unnecessary redundancy in the following descriptions and to facilitate understanding by those skilled in the art. In addition, the accompanying drawings and subsequent descriptions are provided for those skilled in the art to fully understand the present application, and are not intended to limit the subject matter recited in the claims.

The "ranges" disclosed in the present application are defined in the form of lower and upper limits. A given range is defined by selecting a lower limit and an upper limit, and the selected lower and upper limits define the boundaries of the particular range. The range defined in this way may include or may not include end values, and may be arbitrarily combined, that is, any lower limit may be combined with any upper limit to form a range. For example, if the ranges 60-120 and 80-110 are listed for specific parameters, it is understood that the ranges 60-110 and 80-120 are also expected. In addition, if the listed minimum range values are 1 and 2 and if the listed maximum range values are 3, 4, and 5, the following ranges can all be expected: 1-3, 1-4, 1-5, 2-3, 2-4, and 2-5. In the present application, unless otherwise specified, the numerical range "a-b" represents an abbreviated representation of any combination of real numbers between a and b, wherein a and b are both real numbers. For example, the numerical range "0-5" indicates that all real numbers between "0-5" have been listed herein, and "0-5" is only a shortened representation of these numerical combinations. In addition, when a parameter is expressed as an integer greater than or equal to 2, it is equivalent to disclosing that the parameter is, for example, an integer of 2, 3, 4, 5, 6, 7, 8, 9, 10, 11, 12, and the like.

Unless otherwise specified, all embodiments and optional embodiments of the present application may be combined with each other to form new technical solutions.

Unless otherwise specified, all technical features and optional technical features of the present application may be combined with each other to form new technical solutions.

Unless otherwise specified, all steps of the present application may be performed sequentially or randomly, and preferably sequentially. For example, the method includes steps (a) and (b), which means that the method may comprise steps (a) and (b) performed in order, or may comprise steps (b) and (a) performed in order. For example, reference to "the method may further include step (c)" indicates that step (c) may be added to the method in any order, for example, the method may comprise steps (a), (b), and (c), or steps (a), (c), and (b), or steps (c), (a), and (b), etc.

Unless otherwise specifically stated, "including" and "comprising" mentioned in the present application indicate either open inclusion or closed inclusion. For example, the terms "including" and "comprising" may indicate that other components not listed may be further included or comprised, or only the listed components may be included or comprised.

Unless otherwise specifically stated, in the present application, the term "or" is inclusive. For example, the phrase "A or B" means "A, B, or both A and B." More specifically, condition "A or B" is met by either of the following: A is true (or exists) and B is false (or does not exist); A is false (or non-existent) and B is true (or exists); or both A and B are true (or exist).

Formamidinium iodide is one of the important raw materials for perovskite solar cells. With the development of the perovskite solar cells, the demand for formamidinium iodide will continue to increase. In current preparation process, bulk-produced formamidinium iodide products have iodine precipitation and surface yellowing, and the color deepens as the storage time increases. Iodine precipitated from formamidinium iodide will be treated as a carrier recombination center in subsequent preparation of formamidinium iodide perovskite films, thereby reducing the stability and efficiency of the perovskite solar cells.

Therefore, research on the removal of elemental iodine is emerging continuously. For example, formamidinium iodide and a preparation method therefor, and the method includes: mixing methylammonium acetate with hydroiodic acid, evaporating the mixture to obtain a slush-like material, and then heating and calcining the slush-like material to obtain formamidinium iodide. In this method, it does not require washing with solvents such as ether, significantly reducing solvent costs and thereby decreasing the synthesis cost of formamidinium iodide; and however, in this solution, it requires high-temperature calcination, on one hand, it demands uniform temperature distribution, and on the other hand, high temperature may lead to the risk of scald.

Unexpectedly, by adding a reducing agent in the preparation process of formamidinium iodide, elemental iodine in formamidinium iodide can be reduced, and the stability is improved, therefore, the stability and efficiency of the perovskite solar cell are improved, and moreover, the risk of scald caused by high temperature can be avoided.

Based on this, the present application provides application of an additive, formamidinium iodide and a preparation method therefor, a perovskite solar cell, a solar power generation apparatus, and an electrical apparatus.

In a first aspect, the embodiment of the present application provides the application of the additive in preparation of formamidinium iodide, and the additive includes a reducing agent.

According to the technical solution in the embodiment of the present application, by incorporating the reducing agent in the additive and using the additive to prepare formamidinium iodide, the generation of elemental iodine in the preparation process of formamidinium iodide can be reduced; in addition, iodine which is oxidized to elemental iodine can be reduced to iodine anions so as to generate formamidinium iodide, therefore, the stability of formamidinium iodide is improved, and then the stability and efficiency of the perovskite cell are improved.

In any embodiment, the reducing agent includes a compound containing at least one of elements N, O, S and P. When the reducing agent including the compound containing at least one of elements N, O, S and P is used for preparing formamidinium iodide, the generation of the elemental iodine in the preparation process of formamidinium iodide can be reduced, and moreover, iodine which is oxidized to elemental iodine can be reduced to iodine anions so as to generate formamidinium iodide, therefore, the stability of formamidinium iodide is improved; moreover, when the prepared formamidinium iodide is applied to the perovskite cell, in a perovskite precursor solution, and in the presence of the reducing agent including the compound containing at least one of elements N, O, S and P, the reducing agent will be complexed with iodide, thus the solvent will be quickly removed, perovskite can be quickly nucleated and slowly crystallized, and then the stability and efficiency of the perovskite cell are further improved. In absence of the reducing agent including the compound containing at least one of elements N, O, S and P, iodide will be complexed with DMSO in the solvent, so that the crystallization rate of perovskite is increased, which does not facilitate nucleation and solvent volatilization.

In any embodiment, the reducing agent includes at least one of hypophosphorous acid, red phosphorus, sulfurous acid, L-ascorbic acid, sodium thiosulfate, sodium hypophosphite, oxalic acid, hydrosulfuric acid and formamidinium sulfinic acid. By using at least one of the reducing agents, iodine in the preparation process of formamidinium iodide can be further reduced, thus the perovskite nucleation rate is increased, the perovskite crystallization rate is decreased, the solvent volatilization rate is increased, and then the stability and efficiency of the perovskite cell are improved. In some embodiments of the present application, the reducing agent can only include one of hypophosphorous acid, red phosphorus, sulfurous acid, L-ascorbic acid, sodium thiosulfate, sodium hypophosphite, oxalic acid, hydrosulfuric acid and formamidinium sulfinic acid, and in other embodiments of the present application, two or more of them can be included at the same time.

In a second aspect, the embodiment of the present application provides the preparation method for formamidinium iodide, and the additive in the first aspect is used. In some embodiments of the preparation method for formamidinium iodide in the present application, the steps of using the additive are not limited, the additive can be pretreated in raw materials, or the additive can be added in the reaction process to reduce the generation of elemental iodine.

In any embodiment, the preparation method for formamidinium iodide includes the following steps:

mixing hydroiodic acid with the additive, cooling and storing to obtain a hydroiodic acid pretreatment solution; and
mixing a formamidinium acetate solution with the hydroiodic acid pretreatment solution, crystallizing, washing and drying to obtain formamidinium iodide.

The additive is added into hydroiodic acid, and cooled and stored to obtain the hydroiodic acid pretreatment solution; the reducing agent is added into the hydroiodic acid, the reducing agent will react with the elemental iodine in the hydroiodic acid firstly to reduce the elemental iodine to hydroiodic acid; and the reducing agent is oxidized, so that the content of the elemental iodine in the hydroiodic acid pretreatment solution is reduced. Then the hydroiodic acid pretreatment solution reacts with formamidinium acetate, and a main product of the reaction is formamidinium iodide, and therefore, the content of the elemental iodine in the formamidinium iodide is reduced. In addition, the cooling and storing can further reduce the generation of the elemental iodine. If the reducing agent is added in the reaction process, part of the reducing agent will react with formamidinium acetate to consume the reaction raw material formamidinium acetate and generate a side reaction product, on one hand, the reducing agent reacting with the elemental iodine will be reduced, and the content of the elemental iodine will be increased; and on the other hand, the side reaction product will be produced and is not conducive to the stability and cycle performance of the perovskite cell.

In any embodiment, in the step of mixing hydroiodic acid with the additive, cooling and storing to obtain the hydroiodic acid pretreatment solution,
the mass ratio of the additive in the hydroiodic acid pretreatment solution is 0.1%-10%, and the mass ratio of the additive in the hydroiodic acid pretreatment solution is within this range, so that the inhibition effect on the elemental iodine can be improved; the reducing agent is provided to react with the elemental iodine, and meanwhile, the reaction between the reducing agent and the formamidinium acetate is reduced, thus side reaction products can be reduced, and impurities can be reduced; and when the prepared formamidinium iodide is used for preparing the perovskite cell, the stability and the cycle performance of the perovskite cell are facilitated, and the cost is reduced. The mass ratio of the additive in the hydroiodic acid pretreatment solution can be 0.1%, 0.5%, 1%, 1.5%, 2%, 2.5%, 3%, 3.5%, 4%, 4.5%, 5%, 6%, 7%, 8%, 9% or 10%. Optionally, the mass ratio of the additive in the hydroiodic acid pretreatment solution is 0.5%-5%.

In any embodiment, in the step of mixing hydroiodic acid with the additive, cooling and storing to obtain the hydroiodic acid pretreatment solution, the cooling and storing temperature is 0-10°C, which can reduce icing, and is conducive to the reaction between the reducing agent and the elemental iodine, thereby reducing the precipitation of the elemental iodine; the cooling and storing temperature can be 0°C, 1°C, 2°C, 3°C, 4°C, 5°C, 6°C, 7°C, 8°C, 9°C or 10°C; and optionally, the cooling and storing temperature is 0-5°C.

In any embodiment, in the step of mixing hydroiodic acid with the additive, cooling and storing to obtain the hydroiodic acid pretreatment solution, the cooling and storing time is 24-72 h, which can improve the reaction efficiency between the reducing agent and the elemental iodine, as well as improving the production efficiency. The cooling and storing time can be 24 h, 30 h, 36 h, 42 h, 48 h, 54 h, 60 h, 66 h or 72 h; and optionally, the cooling and storing time is 36-60 h.

In any embodiment, in the step of mixing the formamidinium acetate solution with the hydroiodic acid pretreatment solution, crystallizing, washing and drying to obtain formamidinium iodide, the ratio of the amount of substance of formamidinium acetate to hydroiodic acid is 1: (0.9-1.2), appropriate ratio of the amount of substance can ensure sufficient reaction, and reduce the residues of reaction raw materials; and the ratio of the amount of substance of formamidinium acetate to hydroiodic acid can be 1:0.9, 1:0.95, 1:1, 1:1.05, 1:1.06, 1:1.07, 1:1.08, 1:1.09, 1:1.1, 1:1.15 or 1:1.2. Optionally, the ratio of the amount of substance of the formamidinium acetate solution to hydroiodic acid is 1: (1.05-1.1).

In any embodiment, in the step of mixing the formamidinium acetate solution with the hydroiodic acid pretreatment solution, crystallizing, washing and drying to obtain formamidinium iodide, the mixing temperature is 0-35°C, and at this temperature, the yield of formamidinium iodide can be increased, and meanwhile icing is reduced; the mixing temperature can be 0°C, 1°C, 2°C, 3°C, 4°C, 5°C, 8°C, 10°C, 15°C, 20°C, 25°C, 30°C or 35°C; and optionally, the mixing temperature is 0-5°C.

In any embodiment, in the step of mixing the formamidinium acetate solution with the hydroiodic acid pretreatment solution, crystallizing, washing and drying to obtain formamidinium iodide, the mixing time is 2-8 h, at such time, hydroiodic acid and formamidinium acetate can react sufficiently, thereby increasing the yield of formamidinium iodide, making reaction more sufficient, and reducing the influence on the production efficiency; the mixing time can be 2 h, 2.5 h, 3 h, 3.5 h, 4 h, 4.5 h, 5 h, 5.5 h, 6 h, 6.5 h, 7 h, 7.5 h or 8 h; and optionally, the mixing time is 3-5 h. In some embodiments of the present application, the hydroiodic acid pretreatment solution and formamidinium acetate hydroiodic acid can be mixed by slowly adding dropwise the hydroiodic acid pretreatment solution into the formamidinium acetate solution by a constant-pressure funnel, and after adding dropwise is finished and the hydroiodic acid solution is transparent, mixing is carried out to a preset time.

In any embodiment, in the step of mixing the formamidinium acetate solution with the hydroiodic acid pretreatment solution, crystallizing, washing and drying to obtain formamidinium iodide, the crystallization temperature is 0-35°C, which can increase the crystallization rate of formamidinium iodide, and reduce icing; the crystallizing temperature can be 0°C, 1°C, 2°C, 3°C, 4°C, 5°C, 8°C, 10°C, 15°C, 20°C, 25°C, 30°C or 35°C; and optionally, the crystallizing temperature is 0-10°C.

In any embodiment, in the step of mixing the formamidinium acetate solution with the hydroiodic acid pretreatment solution, crystallizing, washing and drying to obtain formamidinium iodide, the drying mode includes vacuum drying, in which, the drying pressure is 0 bar to -1 bar, contact between formamidinium iodide and oxygen can be reduced by vacuum drying, and then the generation of elemental iodine is reduced; the drying pressure can be 0 bar, -0.1 bar, -0.2 bar, -0.3 bar, -0.4 bar, -0.5 bar, -0.6 bar, -0.7 bar, -0.8 bar, -0.9 bar or -1 bar; and optionally, the drying pressure is -0.8 bar to -1 bar.

In any embodiment, in the step of mixing the formamidinium acetate solution with the hydroiodic acid pretreatment solution, crystallizing, washing and drying to obtain formamidinium iodide, the drying temperature is 40-80°C, which is conducive to volatilization of the solvent, and reduces the scalding risk in the preparation process; the drying temperature can be 40°C, 45°C, 50°C, 55°C, 60°C, 62°C, 65°C, 68°C, 70°C, 75°C or 80°C; and optionally, the drying temperature is 60-70°C.

In any embodiment, in the step of mixing the formamidinium acetate solution with the hydroiodic acid pretreatment solution, crystallizing, washing and drying to obtain formamidinium iodide, the drying time is 6-15 h, and if the drying time is less than 6 h, the residues of the solvent can be reduced, and the production efficiency can be improved; the drying time can be 6 h, 7 h, 8 h, 8.5 h, 9 h, 9.5 h, 10 h, 10.5 h, 11 h, 11.5 h, 12 h, 13 h, 14 h or 15 h; and optionally, the drying time is 8-12 h.

In any embodiment, the step of mixing the formamidinium acetate solution with the hydroiodic acid pretreatment solution, crystallizing, washing and drying to obtain formamidinium iodide includes:

mixing the formamidinium acetate solution with the hydroiodic acid pretreatment solution to obtain a mixed solution;
evaporating the mixed solution, and removing the solvent to obtain a formamidinium iodide crude extract; and
mixing the formamidinium iodide crude extract with a solvent, heating, recrystallizing, washing and drying to obtain formamidinium iodide.

By recrystallizing the formamidinium iodide crude extract, impurity residues in the formamidinium iodide crude extract can be further removed, and therefore, the purity of formamidinium iodide is improved. In some embodiments of the present application, recrystallization can be one-time recrystallization or multi-recrystallization according to different requirements.

In some embodiments of the present application, a rotary evaporation mode can be used for evaporating the mixed solution, and the solvent is removed to obtain a white powdery formamidinium iodide solid. In the washing process, glacial ethanol can be adopted as a detergent for washing for multiple times.

In any embodiment, in the step of mixing the formamidinium iodide crude extract with the additive, crystallizing, washing and drying to obtain formamidinium iodide, the solvent includes ethanol, and the ethanol is used as the solvent and is conveniently removed, so that the impurities in formamidinium iodide can be decreased.

In any embodiment, in the step of mixing the formamidinium iodide crude extract with the additive, crystallizing, washing and drying to obtain formamidinium iodide, the heating temperature is 30-70°C, which is conducive to the dissolution of formamidinium iodide, and the volatilization speed of ethanol is controlled, thereby facilitating recrystallization; the heating temperature can be 30°C, 35°C, 40°C, 45°C, 50°C, 55°C, 60°C, 65°C or 70°C; and optionally, the heating temperature is 40-50°C.

In any embodiment, in the step of mixing the formamidinium iodide crude extract with the additive, crystallizing, washing and drying to obtain formamidinium iodide, the recrystallization temperature is 0-35°C, which can increase the crystallization rate of formamidinium iodide and reduce icing; the recrystallization temperature can be 0°C, 1°C, 2°C, 3°C, 4°C, 5°C, 8°C, 10°C, 15°C, 20°C, 25°C, 30°C or 35°C; and optionally, the recrystallization temperature is 0-5°C.

In a third aspect, the embodiment of the present application provides formamidinium iodide which is prepared by the preparation method for formamidinium iodide in the second aspect of the present application.

Whether the reducing agent is added in the preparation process of formamidinium iodide can be detected by detecting the content of anions in formamidinium iodide, and it is to be noted that because of recrystallization in the preparation process, the content of the anions is relatively small, and the content of the anions can be detected by anion chromatography, and thus the detection limit of the anions is improved.

In a fourth aspect, the embodiment of the present application provides a perovskite solar cell which includes formamidinium iodide in the third aspect of the present application.

In some embodiments, the perovskite solar cell can further include a conductive glass layer, an electron transport layer, a perovskite layer, and a hole transport layer, in which, the perovskite layer includes formamidinium iodide in the fourth aspect of the present application.

It is to be noted that formamidinium iodide can exist in the perovskite layer in a form of an $FA_xA'_{1-x}BX_3$ complex, in which, A' refers to a monovalent inorganic cation or an organic ammonium cation or a mixture thereof and can be at least one of methylamine ions (MA) and Cs; B refers to a divalent inorganic metal cation and can be at least one of $Pb^{2+}$ and $Sn^{2+}$; X refers to a halogen element or a halogen-like element and can be at least one of $Cl^-$, $Br^-$, $I^-$, $SCN^-$, $CNO^-$, $OCN^-$, $OSCN^-$, $CP^-$, $CN^-$, $SeCN^-$, $N_3^-$, and $NO_2^-$; and $0<x\leq1$. $FAPbI_3$ is taken as an example. After the perovskite layer is dissolved in water, the $FA_xA'_{1-x}BX_3$ complex can be decomposed to obtain potassium iodide amidine that is dissolved in water, so that it is verified that the perovskite solar cell contains formamidinium iodide. Whether the reducing agent is added in the preparation process of formamidinium iodide can be detected by detecting the content of anions in formamidinium iodide, and it is to be noted that because of recrystallization in the preparation process, the content of the anions is relatively small, and the content of the anions can be detected by anion chromatography, and thus the detection limit of the anions is improved.

In a fifth aspect, the embodiment of the present application provides a solar power generation apparatus which includes the perovskite solar cell in the fourth aspect of the present application.

In some embodiments, the solar power generation apparatus can include a mounting bracket, the perovskite solar cell, a secondary battery and an electric power output portion; and the electric energy generated by the perovskite solar cell through sunlight is stored in the secondary battery and outputted by the electric power output portion for use.

In other embodiments, the solar power generation apparatus can directly output the electric energy, which is generated by the perovskite solar cell through sunlight, by the electric power output portion for use without the secondary battery.

In a sixth aspect, the embodiment of the present application provides an electrical apparatus which includes the solar power generation apparatus in the fifth aspect of the present application.

The electrical apparatus can include, but are not limited to, solar street lights, solar water heaters, solar charging stations, solar-powered satellite ships, and solar trams.

The following further details the technical solutions of the present application with reference to specific embodiments. It is to be understood that the following embodiments are provided merely to explain the present application and are not intended to limit the present application.

**Example 1**

A preparation method for formamidinium iodide included the following steps:
mixing hydroiodic acid with a formamidinium acetate solution in a ratio of the amount of substance of 1:1 at 4°C for 4 h, adding a reducing agent that was hypophosphorous acid, in which, the addition amount of hypophosphorous acid was 0.5% of the total mass of hydroiodic acid and hypophosphorous acid; heating at 45°C, crystallizing at 4°C, and washing with glacial ethanol for 3 times; and performing vacuum drying at 65°C under -0.9 bar for 12 h to obtain formamidinium iodide.

**Example 2**

A preparation method for formamidinium iodide included the following steps:

mixing hydroiodic acid with hypophosphorous acid, and storing at 4°C for 48 h to obtain a hydroiodic acid pretreatment solution, in which, the mass ratio of hypophosphorous acid in the hydroiodic acid pretreatment solution was 0.5%; and mixing a formamidinium acetate solution with the hydroiodic acid pretreatment solution at 4°C for 4 h, heating at 45°C, crystallizing at 4°C, washing with glacial ethanol for 3 times, and performing vacuum drying at 65°C under -0.9 bar for 12 h to obtain formamidinium iodide.

The ratio of amount of substance of hydroiodic acid to formamidinium acetate was 1:1.

Example 3

A preparation method for formamidinium iodide included the following steps:

mixing hydroiodic acid with hypophosphorous acid, and storing at 4°C for 48 h to obtain a hydroiodic acid pretreatment solution, in which, the mass ratio of hypophosphorous acid in the hydroiodic acid pretreatment solution was 0.5%; and

mixing a formamidinium acetate solution with the hydroiodic acid pretreatment solution at 4°C for 4 h to obtain a mixed solution, in which, the ratio of the amount of substance of hydroiodic acid to formamidinium acetate was 1:1;

evaporating the mixed solution at normal temperature, and removing a solvent to obtain a formamidinium iodide crude extract; and

mixing the formamidinium iodide crude extract with ethanol, heating at 45°C, recrystallizing at 4°C, washing with glacial ethanol for 3 times, and performing vacuum drying at 65°C under -0.9 bar for 12 h to obtain formamidinium iodide.

**Example 4**

A preparation method for formamidinium iodide included the following steps:

mixing hydroiodic acid with hypophosphorous acid, and cooling and storing at 4°C for 48 h to obtain a hydroiodic acid pretreatment solution, in which, the mass ratio of hypophosphorous acid in the hydroiodic acid pretreatment solution was 0.5%;

mixing a formamidinium acetate solution with the hydroiodic acid pretreatment solution at 4°C for 4 h to obtain a mixed solution, in which, the ratio of the amount of substance of hydroiodic acid to formamidinium acetate was 1:1;

performing rotary evaporation on the mixed solution, and removing a solvent to obtain a formamidinium iodide crude extract; and

mixing the formamidinium iodide crude extract with ethanol, heating at 45°C, recrystallizing at 4°C, washing with glacial ethanol for 3 times, and performing vacuum drying at 65°C under -0.9 bar for 12 h to obtain formamidinium iodide.

According to the present application, some parameters of the Example 5 to the Example 23 and a Comparative Example 1 to a Comparative Example 2 are set according to Table 1, and other parameters are the same as those of the Example 4.

Table 1 Parameters of additives in Example 5 to Example 23 and Comparative Example 1 to Comparative Example 2

|  | Additive | Mass ratio of additive in hydroiodic acid pretreatment solution | Photoelectric conversion efficiency (%) | Light stability 1000h (%) | Color of formamidinium iodide | Content of anion |
|---|---|---|---|---|---|---|
| Example 5 | Stannous iodide | 1.5% | 19.41% | 19.31% | Faint yellow | 0.02% |
| Example 6 | Stannous chloride | 1.5% | 19.29% | 19.27% | Faint yellow | 0.01% |
| Example 7 | Hypophosphorous acid | 1.5% | 21.97% | 22.01% | Bright white | 0.02% |
| Example 8 | Red phosphorus | 1.5% | 21.65% | 21.55% | Bright white | 0.02% |
| Example 9 | sulfurous acid | 1.5% | 21.82% | 21.68% | Bright white | 0.01% |
| Example 10 | L-ascorbic acid | 1.5% | 21.76% | 21.88% | Bright white | 0.01% |
| Example 11 | Sodium thiosulfate | 1.5% | 20.86% | 20.53% | Bright white | 0.02% |
| Example 12 | Sodium hypophosphite | 1.5% | 20.46% | 20.33% | Bright white | 0.01% |
| Example 13 | Oxalic acid | 1.5% | 20.95% | 20.59% | Bright white | 0.02% |
| Example 14 | hydrosulfuric acid | 1.5% | 21.32% | 21.56% | Bright white | 0.01% |
| Example 15 | Formamidinium sulfinic acid | 1.5% | 20.96% | 20.49% | Bright white | 0.02% |
| Example 16 | Hypophosphorous acid, red phosphorus | 0.75% for each | 21.58% | 21.43% | Bright white | 0.02% |

(continued)

| | Additive | Mass ratio of additive in hydroiodic acid pretreatment solution | Photoelectric conversion efficiency (%) | Light stability 1000h (%) | Color of formamidinium iodide | Content of anion |
|---|---|---|---|---|---|---|
| Example 17 | Sodium hypophosphite, L-ascorbic acid, oxalic acid | 0.5% for each | 20.56% | 20.33% | Bright white | 0.01% |
| Example 18 | Hypophosphorous acid | 0.08% | 19.72% | 19.68% | Faint yellow | 0.01% |
| Example 19 | Hypophosphorous acid | 0.1% | 19.83% | 19.77% | Bright white | 0.02% |
| Example 20 | Hypophosphorous acid | 5% | 20.89% | 20.85% | Bright white | 0.1% |
| Example 21 | Hypophosphorous acid | 7% | 20.56% | 20.47% | Bright white | 0.5% |
| Example 22 | Hypophosphorous acid | 10% | 19.56% | 19.75% | Bright white | 0.7% |
| Example 23 | Hypophosphorous acid | 12% | 19.49% | 19.67% | Bright white | 0.9% |
| Comparative Example 1 | Ammonium persulfate | 1.5% | 17.57% | 11.46% | Reddish brown | 0.02% |
| Comparative Example 2 | - | 0% | 18.23% | 16.86% | Yellow | None |

**Performance test**

**A perovskite solar cell was prepared from formamidinium iodide prepared by the** preparation method for formamidinium iodide in the Example 5 to the Example 23 and the Comparative Example 1 to the Comparative Example 2, the stability performance test was performed, moreover, the color of formamidinium iodide prepared by the preparation method for formamidinium iodide in the Example 1 to the Example 23 and the Comparative Example 1 to the Comparative Example 2 was observed to detect the content of anions in formamidinium iodide.

The preparation process for the perovskite solar cell was as follows:

[Conductive glass]

The conductive glass with a fluorine-doped tin oxide (FTO with a thickness of 40 nm) film was purchased and could be directly used after being cleaned.

[Hole transport layer and passivation layer]

The cleaned conductive glass was irradiated for 10 min by an ultraviolet ozone machine. 50 mg of nickel nitrate hexahydrate was weighed and dissolved in 1 mL of methanol. A supernatant was collected and spin-coated on the conductive glass, and then annealing was carried out according to the following procedures: the conductive glass was kept at the temperature of 80°C for 10 min, the temperature was raised to 345°C within 30 min, then the conductive glass was kept at 345°C for 30 min, and then was taken out after being cooled to 100°C, thus obtaining the hole transport layer (with the thickness of 30 nm); and

passivation layer: a 10 mg/mL potassium chloride aqueous solution was prepared and spin-coated on the hole transport layer, and annealing was carried out at 100°C for 10 min, so as to the passivation layer (with the thickness of 5 nm).

[Perovskite layer]

Preparation of perovskite solution: 650 mg of formamidinium iodide (FAI), 1,800 mg of lead iodide (PbI2), and 50 mg of methylamine chloride (MACl) were dissolved in 1 mL of solvent; the solvent was a mixed solvent of N,N-Dimethylformamide (DMF) and dimethyl sulfoxide (DMSO), in which, the volume ratio of DMF to DMSO was 4:1 (DMF: DMSO); and a supernatant was collected for later use (formamidinium iodide (FAI) was autonomously synthesized, lead iodide (PbI2) and methylamine chloride (MACl) were purchased from Xi'an Polymer Light Technology Corp., and DMF and DMSO were purchased from Sigma company).
Then 60 μL of the supernatant of the perovskite solution was added dropwise on the passivation layer, and spin-coated by a spin coater for 30 s, and then annealing was carried out at 150°C for 1 h to obtain the perovskite layer with the thickness of 500 nm.

[Electron transport layer and buffer layer]

Preparation of a [6,6]-phenyl-C61-methyl butyrate (PCBM, commercially available) solution: concentration: 20 mg/ml, chlorobenzene served as the solvent;

preparation of a bathocuproine (BCP, commercially available) solution: concentration: 0.5 mg/mL, isopropanol served as the solvent;
the prepared 60 μL of PCBM solution was spin-coated on conductive glass having a prepared perovskite layer for 30 sec. Then annealing was carried out at a temperature of 100°C for 10 min, then the conductive glass was taken down from an instrument, and cooled to room temperature to obtain the electron transport layer; and then the 60 μL of BCP solution was spin-coated on the surface of the electron transport layer for 30 sec. After spin-coating, the electron transport layer (with the thickness of 70 nm) and the buffer layer (with the thickness of 3 nm) were obtained.

[Metal electrode]

80 nm silver was evaporated in a vacuum evaporation device at an evaporation rate of 0.1 A/s. After evaporating, the complete perovskite solar cell was obtained.
A standard sunlight (a spectrum of AM 1.5 G and an irradiance of 100 mW/cm2) was emitted based on a standard simulated sunlight to test the photoelectric conversion efficiency of the cell; the photoelectric conversion efficiency of a device illuminated for 1,000 h was tested; and the Photoelectric Conversion Efficiency (PCE) of the device using the perovskite solar cell for 1,000 h was tested under a relative humidity of $50\pm10\%$ RH and a temperature of 25°C, and the PCE was calculated by the following formula:

$$PCE = \frac{V_{oc} \times J_{sc} \times FF}{P_{in}} \times 100\%$$

in which, the PCE refers to the photoelectric conversion efficiency, Voc refers to an open-circuit voltage, Jsc refers to a short-circuit current density, FF refers to a filling factor, and Pin refers to an input power.
The content of anions in the impurity formamidinium iodide could be analyzed by the ion chromatography.
1 g of sample was weighed, 100 g of ultrapure water was added for dissolving and volume fixing, and then detection was carried out by ion chromatography after sample introduction;

ion chromatography leacheate system: KOH (14 mM);
then qualitative analysis was carried out based on a retention time of the test substance and a standard solution; quantitative analysis was carried out according to a standard substance curve; and analytical measurement was carried out by a ThermoFisher Dionex™ ICS-6000 high-voltage ion chromatograph.

The test results were counted, and the results of the Example 5 to the Example 23 and the Comparative Example 1 to the Comparative Example 2 are shown in Table 1. The results of the Example 1 to the Example 4 are shown in Table 2.

Table 2 Performance test results of Example 1 to Example 4

| | Additive | Mass ratio of additive in hydroiodic acid pretreatment solution | Photoelectric conversion efficiency (%) | Light stability 1000h (%) | Color of formamidinium iodide | Content of anion |
|---|---|---|---|---|---|---|
| Example 1 | Hypophosphorous acid | 0.5% | 19.11% | 19.26% | Faint yellow | 0.05% |
| Example 2 | Hypophosphorous acid | 0.5% | 20.19% | 20.27% | Bright white | 0.01% |
| Example 3 | Hypophosphorous acid | 0.5% | 20.27% | 20.31% | Bright white | 0.01% |
| Example 4 | Hypophosphorous acid | 0.5% | 20.35% | 20.55% | Bright white | 0.01% |

As shown in Table 1, according to the Example 5 to the Example 23 and the Comparative Example 1 to the Comparative Example 2, by incorporating the reducing agent in the additive and using the additive to prepare formamidinium iodide, the generation of elemental iodine in the preparation process of formamidinium iodide can be reduced; in addition, iodine which is oxidized to elemental iodine can be reduced to iodine anions so as to generate formamidinium iodide, therefore, the stability of formamidinium iodide is improved, and then the stability and efficiency of the perovskite cell are improved. The light stability and photoelectric conversion efficiency of the high perovskite cell in the Example 7 to the Example 23 are superior to those of the perovskite cell in the Example 5 to the Example 6, and the light stability and photoelectric conversion efficiency of the high perovskite cell in the Example 5 to the Example 6 are superior to those in the Comparative Example 1 to the Comparative Example 2. By adding the oxidizing agent that is ammonium persulfate in the Comparative Example 1, the generation of elemental iodine can be inhibited, generation of elemental iodine can be increased, and then the photoelectric conversion efficiency and light stability are affected. No additive is added in the Comparative Example 2, so the photoelectric conversion efficiency and light stability are lower than those in the Example 5 to the Example 23, and elemental iodine is generated more easily.

According to the Example 5 to the Example 17, when the reducing agent including the compound containing at least one of elements N, O, S and P is used for preparing formamidinium iodide, the generation of the elemental iodine in the preparation process of formamidinium iodide can be reduced, and moreover, iodine which is oxidized to elemental iodine can be reduced to iodine anions so as to generate formamidinium iodide, therefore, the stability of formamidinium iodide is improved; moreover, when the prepared formamidinium iodide is applied to the perovskite cell, in a perovskite precursor solution, and in the presence of the reducing agent including the compound containing at least one of elements N, O, S and P, the reducing agent will be complexed with iodide, thus the solvent will be quickly removed, perovskite can be quickly nucleated and slowly crystallized, and then the stability and efficiency of the perovskite cell are further improved. In absence of the reducing agent including the compound containing at least one of elements N, O, S and P, iodide will be complexed with DMSO in the solvent, so that the crystallization rate of perovskite is increased, which does not facilitate nucleation and solvent volatilization.

According to the Example 7 and the Example 18 to the Example 23, when the mass ratio of the additive in hydroiodic acid pretreatment solution is less than 0.1%, thus the inhibition effect on elemental iodine can be improved, the reaction between the reducing agent and formamidinium acetate is reduced, side reaction products are reduced, impurities are reduced, the light stability and cycle performance of the perovskite cell are facilitated, and the cost is reduced.

As shown in Table 2, in the Example 1, hydroiodic acid and the formamidinium acetate solution were mixed, and then the reducing agent was added; although the generation of elemental iodine could be inhibited by comparing with the Comparative Example 1 and the Comparative Example 2, and the effect was superior to that of the Comparative Example 1 and the Comparative Example 2, the effect was not as good as that of the Example 2 and the Example 23; and the reducing agent was directly added into the hydroiodic acid, on one hand, the generation of the elemental iodine can be reduced, and on the other hand, the reaction between the reducing agent and formamidinium acetate can be reduced, and side reaction products are reduced.

In conclusion, in the additive provided by the present application, by incorporating the reducing agent in the additive and using the additive to prepare formamidinium iodide, the generation of elemental iodine in the preparation process of formamidinium iodide can be reduced; in addition, iodine which is oxidized to elemental iodine can be reduced to iodine anions so as to generate formamidinium iodide, therefore, the stability of formamidinium iodide is improved, and then the stability and efficiency of the perovskite cell are improved.

The above are merely the preferred embodiments of the present application and do not thus limit the patent scope of the present application. For those skilled in the art, various modifications and variations of the present application are possible. Any modifications, equivalent replacements, or improvements made within the spirit and principles of the present application should be included within the scope of patent protection of the present application.

**Claims**

1. Use of an additive in preparation of formamidinium iodide, the additive comprising a reducing agent.

2. The use of an additive in preparation of formamidinium iodide according to claim 1, wherein the reducing agent comprises a compound containing at least one of elements N, O, S and P.

3. The use of an additive in preparation of formamidinium iodide according to claim 2, wherein the reducing agent comprises at least one of hypophosphorous acid, red phosphorus, sulfurous acid, L-ascorbic acid, sodium thiosulfate, sodium hypophosphite, oxalic acid, hydrosulfuric acid and formamidinium sulfinic acid.

4. A preparation method for formamidinium iodide, using the additive according to any one of claims 1 to 3.

5. The preparation method for formamidinium iodide according to claim 4, comprising the following steps:

   mixing hydroiodic acid with the additive, cooling and storing to obtain a hydroiodic acid pretreatment solution; and mixing a formamidinium acetate solution with the hydroiodic acid pretreatment solution, crystallizing, washing and drying to obtain formamidinium iodide.

6. The preparation method for formamidinium iodide according to claim 5, wherein:
   the mass ratio of the additive in the hydroiodic acid pretreatment solution is 0.1%-10%; and optionally, the mass ratio of the additive in the hydroiodic acid pretreatment solution is 0.5%-5%.

7. The preparation method for formamidinium iodide according to claim 5 or 6, wherein:

   the cooling and storing temperature is 0-10°C; optionally, the cooling and storing temperature is 0-5°C; and/or, the cooling and storing time is 24-72 h; and optionally, the cooling and storing time is 36-60 h.

8. The preparation method for formamidinium iodide according to any one of claims 5 to 7, wherein in the step of mixing the formamidinium acetate solution with the hydroiodic acid pretreatment solution,
   the ratio of the amount of substance of formamidinium acetate to hydroiodic acid is 1: (0.9-1.2); and optionally, the ratio of the amount of substance of formamidinium acetate to hydroiodic acid is 1: (1.05-1.1).

9. The preparation method for formamidinium iodide according to any one of claims 5 to 8, wherein in the step of mixing the formamidinium acetate solution with the hydroiodic acid pretreatment solution,

   the mixing temperature is 0-35°C; optionally, the mixing temperature is 0-5°C; and/or,
   the mixing time is 2-8 h; optionally, the mixing time is 3-5 h; and/or,
   the crystallization temperature is 0-35°C; optionally, the crystallization temperature is 0-10°C; and/or,
   the drying mode comprises vacuum drying, in which, the drying pressure is 0 bar to -1 bar; optionally, the drying pressure is -0.8 bar to -1 bar; and/or,
   the drying temperature is 40-80°C; optionally, the drying temperature is 60-70°C; and/or,
   the drying time is 6-15 h; and optionally, the drying time is 8-12 h.

10. The preparation method for formamidinium iodide according to any one of claims 5 to 9, wherein the step of mixing the formamidinium acetate solution with the hydroiodic acid pretreatment solution, crystallizing, washing and drying to obtain formamidinium iodide comprises:

    mixing the formamidinium acetate solution with the hydroiodic acid pretreatment solution to obtain a mixed solution;
    evaporating the mixed solution, and removing the solvent to obtain a formamidinium iodide crude extract; and mixing the formamidinium iodide crude extract with a solvent, heating, recrystallizing, washing and drying to obtain formamidinium iodide.

11. The preparation method for formamidinium iodide according to claim 10, wherein:

the solvent comprises ethanol; and/or,
the heating temperature is 30-70°C; optionally, the heating temperature is 40-50°C; and/or,
the recrystallization temperature is 0-35°C; and optionally, the recrystallization temperature is 0-5°C.

12. Formamidinium iodide, being prepared by the preparation method for formamidinium iodide according to any one of claims 4 to 11.

13. A perovskite solar cell, comprising formamidinium iodide according to claim 12.

14. A solar power generation apparatus, comprising the perovskite solar cell according to claim 13.

15. An electrical apparatus, comprising the solar power generation apparatus according to claim 14.

## INTERNATIONAL SEARCH REPORT

| International application No. |
|---|
| **PCT/CN2024/102626** |

### A. CLASSIFICATION OF SUBJECT MATTER

C07C 257/12(2006.01)i

According to International Patent Classification (IPC) or to both national classification and IPC

### B. FIELDS SEARCHED

Minimum documentation searched (classification system followed by classification symbols)

IPC: C07C

Documentation searched other than minimum documentation to the extent that such documents are included in the fields searched

Electronic data base consulted during the international search (name of data base and, where practicable, search terms used)

CNTXT: ENTXTC; VEN; CNABS; ENTXT; CNKI; ISI web of science; 万方, WANFANG: 宁德时代新能源科技股份有限公司, 栾博, 梁伟风, 史若璇, 昌宇航, 郭永胜, 陈国栋, 碘化甲脒, 碘甲脒, 甲脒氢碘酸, 氢碘酸甲脒, 甲脒氢碘盐酸盐, 氢碘化甲脒, 醋酸甲脒碘化物, 甲酰胺氢碘化物, 还原剂, 次磷酸, 红磷, 亚硫酸, 抗坏血酸, 硫代硫酸钠, 次磷酸钠, 草酸, 氢硫酸, 甲脒亚磺酸, 维生素C, H3PO2, P, H2SO3, NaH2PO2, Na2S2O3, H2C2O4, H2S, formamidine iodide, reducing, hypophosphorous acid, red phosphorous, sulfurous acid, ascorbate sodium thiosulfate, sodium hypophosphite, oxalic acid, hydrogen sulfate, formamidinesulfinic acid

### C. DOCUMENTS CONSIDERED TO BE RELEVANT

| Category* | Citation of document, with indication, where appropriate, of the relevant passages | Relevant to claim No. |
|---|---|---|
| X | CN 115806507 A (HUNAN YITAI TECHNOLOGY CO., LTD.) 17 March 2023 (2023-03-17) description, paragraphs [0002]-[0016] | 1-15 |
| A | CN 115894295 A (WUXI JIDIAN LIGHT ENERGY TECHNOLOGY CO., LTD.) 04 April 2023 (2023-04-04) entire document | 1-15 |
| A | CN 110105246 A (SVOLT ENERGY TECHNOLOGY CO., LTD.) 09 August 2019 (2019-08-09) entire document | 1-15 |
| A | CN 111153808 A (HANGZHOU MICROQUANTA CO., LTD.) 15 May 2020 (2020-05-15) entire document | 1-15 |
| A | CN 109713133 A (NANKAI UNIVERSITY) 03 May 2019 (2019-05-03) entire document | 1-15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **12 September 2024** | **26 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/102626**

| Patent document cited in search report | | | Publication date (day/month/year) | Patent family member(s) | | | Publication date (day/month/year) |
|---|---|---|---|---|---|---|---|
| CN | 115806507 | A | 17 March 2023 | CN | 115806507 | B | 29 March 2024 |
| CN | 115894295 | A | 04 April 2023 | None | | | |
| CN | 110105246 | A | 09 August 2019 | None | | | |
| CN | 111153808 | A | 15 May 2020 | None | | | |
| CN | 109713133 | A | 03 May 2019 | CN | 109713133 | B | 10 May 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310812876